# EUROPEAN PATENT APPLICATION

(11) **EP 4 434 517 A1**
(43) Date of publication of application: **25.09.2024**
(21) Application number: 22900498.1
(22) Date of filing: 30.11.2022
(51) Int. Cl.: A61K 9/19, A61K 39/395, A61P 19/10

(54) **ANTI-SOST ANTIBODY PHARMACEUTICAL COMPOSITION AND USE THEREOF**

(30) Priority: 30.11.2021 CN 202111462784
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: SHAN, Shuang, Shanghai 200245 (CN); TIAN, Chenmin, Shanghai 200245 (CN)
(74) Representative: Potter Clarkson
(86) International application number: PCT/CN2022/135247
(87) International publication number: WO 2023/098694

(57) **Abstract**

The present invention relates to an anti-SOST antibody pharmaceutical composition and a use thereof. In particular, the present invention relates to a pharmaceutical composition comprising an anti-SOST antibody and an antioxidant, and a use thereof for treating SOST-related diseases or disorders.

## Description

### TECHNICAL FIELD

The disclosure belongs to the field of pharmaceutical formulations and particularly relates to a pharmaceutical composition comprising an anti-SOST antibody and use thereof as a medicament.

### BACKGROUND

The statements herein merely provide background information related to the disclosure and do not necessarily constitute the prior art.

Osteoporosis (OP), including postmenopausal osteoporosis (PMO) and senile osteoporosis, is a systemic metabolic bone disorder characterized by low bone mass and bone microarchitecture deterioration, which leads to decreased bone strength, increased bone fragility, and increased susceptibility to fractures.

Sclerostin (SOST for short), as a new biological target, is used for drug development. It treats osteoporosis by regulating osteoblast anabolism. Sclerostin is a secreted glycoprotein expressed by the SOST gene, the amino acid sequence of which is structurally characterized by 190 residues and a loop domain with cysteines. SOST has been shown to be expressed primarily in osteocytes, with very low levels of expression in osteoblasts, cartilage, liver, kidney, bone marrow, heart, pancreas, etc. Studies have shown that sclerostin inhibits Wnt signaling by binding to the low-density lipoprotein receptor LRP5/6, thereby regulating osteogenesis. At present, monoclonal antibody drugs developed against this target have entered clinical research, and clinical data have confirmed the safety and efficacy of this target in treating osteoporosis by regulating bone metabolism.

WO2016145961A1, among others, discloses anti-SOST antibodies for treating osteoporosis. However, due to the large molecular weights and complex structures of antibody drugs, it is especially important to develop stable formulations for antibody drugs so that their therapeutic effects are ensured.

### SUMMARY

The researchers of the disclosure found through extensive research that where the anti-SOST antibody formulation disclosed in WO2019020069A1 (formula: 10 mM pH 5.0 acetic acid-sodium acetate buffer, 100 mg/mL Ab-5 antibody, 80 mg/mL α,α-trehalose dihydrate, 0.4 mg/mL polysorbate 80, and 4.5 mM calcium chloride) is stored for a long period, some Ab-5 antibody molecules will break at the amino acid residue at position 4 of the heavy chain HCDR3 (set forth in SEQ ID NO: 5), producing fragments that affect the binding of the Ab-5 antibody to the antigen. The researchers of the disclosure surprisingly found through extensive research that the addition of an antioxidant to the aforementioned formulation can effectively reduce the occurrence of breaking within the HCDR3 region of the Ab-5 antibody. Therefore, the present disclosure provides an anti-SOST antibody pharmaceutical composition that can better prevent the Ab-5 antibody from breaking in the HCDR3 region and is thus more stable.

Specifically, the disclosure provides a pharmaceutical composition, comprising an anti-SOST antibody and an antioxidant, wherein the anti-SOST antibody comprises an antibody heavy chain variable region and a light chain variable region, wherein the heavy chain variable region comprises a HCDR1 set forth in SEQ ID NO: 3, a HCDR2 set forth in SEQ ID NO: 9 or 4, and a HCDR3 set forth in SEQ ID NO: 5; and the light chain variable region comprises a LCDR1 set forth in SEQ ID NO: 6, a LCDR2 set forth in SEQ ID NO: 7, and a LCDR3 set forth in SEQ ID NO: 8.

In some embodiments of the pharmaceutical composition, the heavy chain variable region of the anti-SOST antibody comprises a HCDR1 set forth in SEQ ID NO: 3, a HCDR2 set forth in SEQ ID NO: 9, and a HCDR3 set forth in SEQ ID NO: 5, and the light chain variable region comprises a LCDR1 set forth in SEQ ID NO: 6, a LCDR2 set forth in SEQ ID NO: 7, and a LCDR3 set forth in SEQ ID NO: 8.

In some embodiments of the pharmaceutical composition, the anti-SOST antibody may be selected from the group consisting of a murine antibody, a chimeric antibody, and a humanized antibody; in some embodiments, the anti-SOST antibody is a humanized antibody.

In some embodiments of the pharmaceutical composition, the heavy chain variable region of the anti-SOST antibody comprises the amino acid sequence of SEQ ID NO: 10, 11, or 12, and the light chain variable region comprises the amino acid sequence of SEQ ID NO: 13, 14, or 15. In some embodiments, the heavy chain variable region of the anti-SOST antibody comprises the amino acid sequence of SEQ ID NO: 10, and the light chain variable region comprises the amino acid sequence of SEQ ID NO: 13.

In some embodiments of the pharmaceutical composition, the light chain amino acid sequence of the anti-SOST antibody has at least 85% (e.g., 85%, 86%, 87%, 88%, 89%, 90%, 91%, 92%, 93%, 94%, 95%, 96%, 97%, 98%, 99%, or 100%) sequence identity to the light chain of the antibody Ab-5 (SEQ ID NO: 25), and the heavy chain amino acid sequence of the anti-SOST antibody has at least 85% (e.g., 85%, 86%, 87%, 88%, 89%, 90%, 91%, 92%, 93%, 94%, 95%, 96%, 97%, 98%, 99%, or 100%) sequence identity to the heavy chain of the antibody Ab-5 (SEQ ID NO: 22); in some embodiments, the heavy chain of the anti-SOST antibody comprises the amino acid sequence of SEQ ID NO: 22, and the light chain comprises the amino acid sequence of SEQ ID NO: 25.

In some embodiments of the pharmaceutical composition, the antioxidant may be selected from the group consisting of: free radical scavengers (e.g., ascorbic acid, butylated hydroxytoluene (BHT), butylated hydroxyanisole (BHA), sodium sulfite, p-aminobenzoic acid, glutathione, and propyl gallate), chelating agents (e.g., ethylenediaminetetraacetic acid (EDTA), diethylenetriaminepentaacetic acid (DTPA), nitrilotriacetic acid (NTA), N-2-acetamido-2-iminodiacetic acid (ADA), bis(aminoethyl)glycol ether, N,N,N',N'-tetraacetic acid (EGTA), trans-diaminocyclohexanetetraacetic acid (DCTA), glutamic acid and aspartic acid, N-hydroxyethyliminodiacetic acid (HIMDA), N,N-bis-hydroxyethylglycine (bicine) and N-tris(hydroxymethyl)methyl)glycine (tricine), glycylglycine, sodium deoxycholate, ethylenediamine, propylenediamine, diethylenetriamine, triethylenetetraamine (trien), EDTA disodium salt, calcium EDTA oxalic acid, malate, citric acid, citric acid monohydrate, trisodium citrate dihydrate, 8-hydroxyquinolinate, and amino acids), chain terminators (e.g., methionine and cysteine), and the like. In some embodiments, the antioxidant is selected from the group consisting of: methionine, histidine, arginine, EDTA, citric acid, ascorbic acid, butylated hydroxytoluene, butylated hydroxyanisole, sodium sulfite, p-aminobenzoic acid, glutathione, propyl gallate, ethanol, and benzyl alcohol; in some embodiments, the antioxidant is selected from the group consisting of: methionine, histidine, and arginine; in some embodiments, the antioxidant is methionine. In some embodiments of the pharmaceutical composition, the concentration of the antioxidant is 1 mM to 100 mM. In some embodiments, the concentration of the antioxidant is 5 mM to 50 mM. In some embodiments, the concentration of the antioxidant is 5 mM to 25 mM. In some embodiments, the concentration of the antioxidant is 10 mM to 25 mM. In some embodiments, the concentration of the antioxidant is 5 mM to 15 mM. In some embodiments, the concentration of the antioxidant is about 10 mM. In some embodiments, the concentration of the antioxidant is about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 18 mM, about 20 mM, about 22 mM, about 24 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM, or any range between these point values. The term "about" best refers to a range of ±10%; for example, the concentration of the antioxidant is about 10 mM, which means that the concentration of the antioxidant is 10 mM ± 1 mM.

In some embodiments of the pharmaceutical composition, the concentration of the anti-SOST antibody is 1 mg/mL to 300 mg/mL; in some embodiments, the concentration of the anti-SOST antibody is 60 mg/mL to 250 mg/mL; in some embodiments, the concentration of the anti-SOST antibody is 80 mg/mL to 200 mg/mL; in some embodiments, the concentration of the anti-SOST antibody is 80 mg/mL to 180 mg/mL; in some embodiments, the concentration of the anti-SOST antibody is 80 mg/mL to 150 mg/mL; in some embodiments, the concentration of the anti-SOST antibody is 50 mg/mL to 200 mg/mL; in some embodiments, the concentration of the anti-SOST antibody is 50 mg/mL to 150 mg/mL; in some embodiments, the concentration of the anti-SOST antibody is 100 mg/mL to 200 mg/mL; in some embodiments, the concentration of the anti-SOST antibody is 100 mg/mL to 150 mg/mL; in some embodiments, the concentration of the anti-SOST antibody is 60 mg/mL to 120 mg/mL; in some embodiments, the concentration of the anti-SOST antibody is 80 mg/mL to 120 mg/mL; in some embodiments, the concentration of the anti-SOST antibody is 90 mg/mL to 110 mg/mL; in some embodiments, the concentration of the anti-SOST antibody is about 100 mg/mL; in some embodiments, the concentration of the anti-SOST antibody is about 150 mg/mL. In some embodiments, the concentration of the anti-SOST antibody is about 1 mg/mL, about 10 mg/mL, about 20 mg/mL, about 30 mg/mL, about 40 mg/mL, about 50 mg/mL, about 60 mg/mL, about 65 mg/mL, about 70 mg/mL, about 75 mg/mL, about 80 mg/mL, about 85 mg/mL, about 90 mg/mL, about 91 mg/mL, about 92 mg/mL, about 93 mg/mL, about 94 mg/mL, about 95 mg/mL, about 96 mg/mL, about 97 mg/mL, about 98 mg/mL, about 99 mg/mL, about 100 mg/mL, about 101 mg/mL, about 102 mg/mL, about 103 mg/mL, about 104 mg/mL, about 105 mg/mL, about 106 mg/mL, about 107 mg/mL, about 108 mg/mL, about 109 mg/mL, about 110 mg/mL, about 115 mg/mL, about 120 mg/mL, about 125 mg/mL, about 130 mg/mL, about 135 mg/mL, about 140 mg/mL, about 145 mg/mL, about 150 mg/mL, about 155 mg/mL, about 160 mg/mL, about 165 mg/mL, about 170 mg/mL, about 175 mg/mL, about 180 mg/mL, about 185 mg/mL, about 190 mg/mL, about 195 mg/mL, about 200 mg/mL, about 205 mg/mL, about 210 mg/mL, about 215 mg/mL, about 220 mg/mL, about 225 mg/mL, about 230 mg/mL, about 240 mg/mL, about 250 mg/mL, about 260 mg/mL, about 270 mg/mL, about 280 mg/mL, about 290 mg/mL or about 300 mg/mL, or any range between these point values. The term "about" best refers to a range of ±10%; for example, the best protein concentration of the antioxidant is about 100 mg/mL, i.e., 100 mg/mL ± 10 mg/mL.

In some embodiments of the pharmaceutical composition, the pharmaceutical composition has a pH of 4.8 to 5.5; in some embodiments, the pharmaceutical composition has a pH of 5.0 to 5.5; in some embodiments, the pharmaceutical composition has a pH of 4.8 to 5.2; in some embodiments, the pharmaceutical composition has a pH of 5.0 to 5.2; in some embodiments, the pharmaceutical composition has a pH of about 5.0. In some embodiments, the pharmaceutical composition has a pH of about 4.8, about 4.9, about 5.0, about 5.1, about 5.2, about 5.3, about 5.4 or about 5.5, or any range between these point values. The term "about" for these pH values refers to 0.05; for example, about 5.0 means 5.0 ± 0.05.

In some embodiments of the pharmaceutical composition, the pharmaceutical composition further comprises a buffer; in some embodiments, the buffer is selected from the group consisting of an acetate buffer, a histidine buffer, a phosphate buffer, and a succinate buffer; in some embodiments, the buffer is selected from the group consisting of an acetate buffer or a histidine buffer; in some embodiments, the buffer is selected from an acetate buffer; in some embodiments, the buffer is an acetic acid-sodium acetate buffer. In some embodiments, the concentration of the buffer is 1 mM to 30 mM; in some embodiments, the concentration of the buffer is 5 mM to 20 mM; in some embodiments, the concentration of the buffer is 10 mM to 20 mM; in some embodiments, the concentration of the buffer is about 10 mM; in some embodiments, the concentration of the buffer is about 1 mM, about 5 mM, about 10 mM, about 12 mM, about 14 mM, about 15 mM, about 16 mM, about 18 mM, about 20 mM, about 21 mM, about 22 mM, about 23 mM, about 24 mM, about 25 mM, about 26 mM, about 27 mM, about 28 mM, about 29 mM or about 30 mM, or any range between these point values. The term "about" best refers to a range of ±10%; for example, the concentration of the acetic acid-sodium acetate buffer is about 10 mM, i.e., 10 mM ± 1 mM.

In some embodiments of the pharmaceutical composition, the pharmaceutical composition further comprises a sugar; in some embodiments, the sugar is selected from the group consisting of a monosaccharide, a disaccharide, a trisaccharide, a polysaccharide, and a sugar alcohol; in some embodiments, the sugar is selected from the group consisting of a reducing sugar and a non-reducing sugars; in some embodiments, the sugar is selected from the group consisting of glucose, sucrose, trehalose, lactose, fructose, maltose, dextran, glycerin, erythritol, glycerol, arabitol, xylitol, sorbitol, mannitol, melibiose, melezitose, raffinose, mannotriose, stachyose, maltose, lactulose, maltulose, glucitol, maltitol, lactitol, and iso-maltulose; in some embodiments, the sugar is selected from a non-reducing disaccharide; in some embodiments, the sugar is selected from the group consisting of trehalose or sucrose; in some embodiments, the sugar is selected from trehalose (e.g., α,α-trehalose dihydrate); in some embodiments, the sugar is selected from sucrose. In some embodiments, the concentration of the sugar is 40 mg/mL to 95 mg/mL; in some embodiments, the concentration of the sugar is 60 mg/mL to 90 mg/mL; in some embodiments, the concentration of the sugar is about 75 mg/mL; in some embodiments, the concentration of the sugar is about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, or about 95 mg/mL, or any range between these point values. The term "about" best refers to a range of ±10%; for example, the concentration of trehalose or sucrose is about 75 mg/mL, i.e., 75 mg/mL ± 7.5 mg/mL.

In some embodiments, the sugar is used as an osmotic pressure regulator; in some embodiments, the pharmaceutical composition has an osmotic pressure of 270 mOsm to 330 mOsm; in some embodiments, the pharmaceutical composition has an osmotic pressure of 280 mOsm to 320 mOsm; in some embodiments, the osmotic pressure of the pharmaceutical composition is controlled at 290 mOsm to 310 mOsm; in some embodiments, the pharmaceutical composition has an osmotic pressure of about 270 mOsm, about 280 mOsm, about 290 mOsm, about 295 mOsm, about 300 mOsm, about 305 mOsm, about 310 mOsm, about 320 mOsm, or about 330 mOsm, and any range between these points. In some embodiments, the pharmaceutical composition has an osmotic pressure of about 300 mOsm.

In some embodiments of the pharmaceutical composition, the pharmaceutical composition further comprises a viscosity modifier; in some embodiments, the viscosity modifier is selected from the group consisting of a calcium salt, sodium chloride, magnesium chloride, and arginine hydrochloride; in some embodiments, the viscosity modifier is selected from the group consisting of calcium chloride or calcium acetate; in some embodiments, the viscosity modifier is selected from calcium chloride. In some embodiments, the concentration of the viscosity modifier is 1 mM to 150 mM; in some embodiments, the concentration of the viscosity modifier is 1 mM to 20 mM; in some embodiments, the concentration of the viscosity modifier is 1 mM to 10 mM; in some embodiments, the concentration of the viscosity modifier is 2 mM to 10 mM; in some embodiments, the concentration of the viscosity modifier is 3 mM to 6 mM; in some embodiments, the concentration of the viscosity modifier is 4.5 mM to 20 mM; in some embodiments, the concentration of the viscosity modifier is 4.5 mM to 10 mM; in some embodiments, the concentration of the viscosity modifier is about 4.5 mM. In some embodiments, the concentration of the viscosity modifier is about 1 mM, about 2 mM, about 3 mM, about 3.5 mM, about 4 mM, about 4.5 mM, about 5 mM, about 5.5 mM, about 6 mM, about 7 mM, about 8 mM, about 9 mM, about 10 mM, about 15 mM, about 20 mM, about 30 mM, about 60 mM, about 90 mM or about 150 mM, or any range between these point values. The term "about" best refers to a range of ± 10%; for example, the concentration of calcium chloride or calcium acetate is about 4.5 mM, i.e., 4.5 mM ± 0.45 mM.

In some embodiments of the pharmaceutical composition, the pharmaceutical composition further comprises a surfactant. In some embodiments, the surfactant is a nonionic surfactant; in some embodiments, the surfactant is selected from the group consisting of poloxamer (e.g., poloxamer 188), polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like; in some embodiments, the surfactant is polysorbate or poloxamer; in some embodiments, the surfactant is polysorbate 80. In some embodiments, the concentration of the surfactant is 0.02 mg/mL to 0.8 mg/mL; in some embodiments, the concentration of the surfactant is 0.2 mg/mL to 0.6 mg/mL; in some embodiments, the concentration of the surfactant is 0.3 mg/mL to 0.6 mg/mL; in some embodiments, the concentration of the surfactant is about 0.4 mg/mL; in some embodiments, the concentration of the surfactant is about 0.02 mg/mL, about 0.1 mg/mL, about 0.2 mg/mL, about 0.3 mg/mL, about 0.4 mg/mL, about 0.5 mg/mL, about 0.6 mg/mL, about 0.7 mg/mL, or about 0.8 mg/mL, or any range between these point values. The term "about" best refers to a range of ± 10%; for example, the concentration of polysorbate 80 is about 0.4 mg/mL, i.e., 0.4 mg/mL ± 0.04 mg/mL. In some embodiments of the pharmaceutical composition, the pharmaceutical composition comprises: (a) 1 mg/mL to 300 mg/mL anti-SOST antibody; preferably, 50 mg/mL to 200 mg/mL anti-SOST antibody, (b) 1 mM to 100 mM methionine, histidine, or arginine, (c) 40 mg/mL to 95 mg/mL trehalose or sucrose, (d) 1 mM to 30 mM; preferably, 5 mM to 20 mM acetate buffer or histidine buffer, (e) 1 mM to 20 mM; preferably, 4.5 mM to 20 mM; more preferably, 2 mM to 10 mM; and most preferably, 3 mM to 6 mM calcium salt, and (f) 0.02 mg/mL to 0.8 mg/mL polysorbate, and the pharmaceutical composition has a pH of 4.8 to 5.5.

In some embodiments of the pharmaceutical composition, (a) 1 mg/mL to 300 mg/mL said anti-SOST antibody, (b) 1 mM to 100 mM methionine, histidine, or arginine, (c) 40 mg/mL to 95 mg/mL trehalose or sucrose, (d) 1 mM to 30 mM acetate buffer or histidine buffer, (e) 4.5 mM to 20 mM calcium salt, and (f) 0.02 mg/mL to 0.8 mg/mL polysorbate, and the pharmaceutical composition has a pH of 4.8 to 5.5.

In some embodiments of the pharmaceutical composition, the pharmaceutical composition comprises: (a) 1 mg/mL to 150 mg/mL anti-SOST antibody; (b) 1 mM to 100 mM methionine, histidine, or arginine, (c) 40 mg/mL to 95 mg/mL trehalose or sucrose, (d) 1 mM to 30 mM acetate buffer or histidine buffer, (e) 4.5 mM to 20 mM calcium salt, and (f) 0.02 mg/mL to 0.8 mg/mL polysorbate, and the pharmaceutical composition has a pH of 4.8 to 5.5.

In some embodiments of the pharmaceutical composition, (a) 50 mg/mL to 200 mg/mL anti-SOST antibody comprising a heavy chain of SEQ ID NO: 22 and a light chain of SEQ ID NO: 25, (b) 5 mM to 25 mM methionine, (c) 60 mg/mL to 90 mg/mL trehalose or sucrose, (d) 10 mM to 20 mM acetate buffer, (e) 4.5 mM to 10 mM calcium chloride, and (f) 0.3 mg/mL to 0.6 mg/mL polysorbate 80, and the pharmaceutical composition has a pH of 5.0 to 5.2.

In some embodiments of the pharmaceutical composition, (a) 50 mg/mL to 150 mg/mL anti-SOST antibody comprising a heavy chain of SEQ ID NO: 22 and a light chain of SEQ ID NO: 25, (b) 5 mM to 25 mM methionine, (c) 60 mg/mL to 90 mg/mL trehalose or sucrose, (d) 5 mM to 20 mM acetate buffer, (e) 1 mM to 10 mM calcium chloride, and (f) 0.3 mg/mL to 0.6 mg/mL polysorbate 80, and the pharmaceutical composition has a pH of 4.8 to 5.2. The trehalose is preferably α,α-trehalose dihydrate.

In some embodiments of the pharmaceutical composition, the pharmaceutical composition comprises: (a) 80 mg/mL to 120 mg/mL anti-SOST antibody comprising a heavy chain of SEQ ID NO: 22 and a light chain of SEQ ID NO: 25, (b) 5 mM to 25 mM methionine, (c) 60 mg/mL to 90 mg/mL trehalose or sucrose, (d) 10 mM to 20 mM acetate buffer, (e) 4.5 mM to 10 mM calcium chloride, and (f) 0.3 mg/mL to 0.6 mg/mL polysorbate 80, and the pharmaceutical composition has a pH of 5.0 to 5.2.

In some embodiments of the pharmaceutical composition, the pharmaceutical composition comprises: (a) 80 mg/mL to 120 mg/mL anti-SOST antibody comprising a heavy chain of SEQ ID NO: 22 and a light chain of SEQ ID NO: 25, (b) 5 mM to 15 mM methionine, (c) 60 mg/mL to 90 mg/mL trehalose or sucrose, (d) 5 mM to 20 mM acetate buffer, (e) 1 mM to 10 mM calcium chloride, and (f) 0.3 mg/mL to 0.6 mg/mL polysorbate 80, and the pharmaceutical composition has a pH of 4.8 to 5.2.

In some embodiments of the pharmaceutical composition, the pharmaceutical composition comprises: (a) 80 mg/mL to 100 mg/mL anti-SOST antibody comprising a heavy chain of SEQ ID NO: 22 and a light chain of SEQ ID NO: 25, (b) about 10 mM methionine, (c) 70 mg/mL to 80 mg/mL trehalose (e.g., α,α-trehalose dihydrate) or sucrose, (d) about 10 mM acetate buffer, (e) about 4.5 mM calcium chloride, and (f) 0.3 mg/mL to 0.6 mg/mL polysorbate 80, and the pharmaceutical composition has a pH of 4.8 to 5.2.

In some embodiments of the pharmaceutical composition, the pharmaceutical composition comprises: (a) 80 mg/mL to 100 mg/mL anti-SOST antibody comprising a heavy chain of SEQ ID NO: 22 and a light chain of SEQ ID NO: 25, (b) about 10 mM methionine, (c) 70 mg/mL to 80 mg/mL trehalose (e.g., α,α-trehalose dihydrate) or sucrose, (d) about 10 mM acetate buffer, (e) about 4.5 mM calcium chloride, and (f) 0.3 mg/mL to 0.6 mg/mL polysorbate 80, and the pharmaceutical composition has a pH of 5.0 to 5.2.

In some embodiments of the pharmaceutical composition, the pharmaceutical composition comprises: (a) about 100 mg/mL anti-SOST antibody comprising a heavy chain of SEQ ID NO: 22 and a light chain of SEQ ID NO: 25, (b) about 10 mM methionine, (c) about 75 mg/mL trehalose (e.g., α,α-trehalose dihydrate) or sucrose, (d) 10 mM to 20 mM acetic acid-sodium acetate buffer, (e) about 4.5 mM calcium chloride, and (f) about 0.4 mg/mL polysorbate 80, and the pharmaceutical composition has a pH of about 5.0.

In some embodiments of the pharmaceutical composition, the pharmaceutical composition comprises: (a) about 100 mg/mL anti-SOST antibody comprising a heavy chain of SEQ ID NO: 22 and a light chain of SEQ ID NO: 25, (b) about 10 mM methionine, (c) about 75 mg/mL sucrose, (d) about 10 mM acetic acid-sodium acetate buffer, (e) about 4.5 mM calcium chloride, and (f) about 0.4 mg/mL polysorbate 80, and the pharmaceutical composition has a pH of about 5.0.

In some embodiments of the pharmaceutical composition, the pharmaceutical composition comprises: (a) 100 mg/mL ± 10 mg/mL anti-SOST antibody comprising a heavy chain of SEQ ID NO: 22 and a light chain of SEQ ID NO: 25, (b) 10 mM ± 1 mM methionine, (c) 75 mg/mL ± 7.5 mg/mL sucrose, (d) 10 mM ± 1 mM acetic acid-sodium acetate buffer, (e) 4.5 mM ± 0.45 mM calcium chloride, and (f) 0.4 mg/mL ± 0.04 mg/mL polysorbate 80, and the pharmaceutical composition has a pH of 5.0 ± 0.05.

In some embodiments of the pharmaceutical composition, the pharmaceutical composition comprises: (a) 150 mg/mL anti-SOST antibody comprising a heavy chain of SEQ ID NO: 22 and a light chain of SEQ ID NO: 25, (b) about 10 mM methionine, (c) about 75 mg/mL sucrose, (d) about 10 mM acetic acid-sodium acetate buffer, (e) about 4.5 mM calcium chloride, and (f) about 0.4 mg/mL polysorbate 80, and the pharmaceutical composition has a pH of about 5.0.

In some embodiments of the pharmaceutical composition, the pharmaceutical composition comprises: (a) about 100 mg/mL anti-SOST antibody comprising a heavy chain of SEQ ID NO: 22 and a light chain of SEQ ID NO: 25, (b) about 50 mM methionine, (c) about 70 mg/mL sucrose or trehalose (e.g., α,α-trehalose dihydrate), (d) about 10 mM acetic acid-sodium acetate buffer, (e) about 4.5 mM calcium chloride, and (f) about 0.4 mg/mL polysorbate 80, and the pharmaceutical composition has a pH of about 5.0.

In some embodiments of the pharmaceutical composition, the pharmaceutical composition comprises: (a) about 100 mg/mL anti-SOST antibody comprising a heavy chain of SEQ ID NO: 22 and a light chain of SEQ ID NO: 25, (b) about 5 mM, about 10 mM, or about 25 mM methionine, (c) about 80 mg/mL trehalose (e.g., α,α-trehalose dihydrate), (d) about 10 mM acetic acid-sodium acetate buffer, (e) about 4.5 mM calcium chloride, and (f) about 0.4 mg/mL polysorbate 80, and the pharmaceutical composition has a pH of about 5.0.

In some embodiments of the pharmaceutical composition, the pharmaceutical composition comprises: (a) about 50 mg/mL anti-SOST antibody comprising a heavy chain of SEQ ID NO: 22 and a light chain of SEQ ID NO: 25, (b) about 10 mM methionine, (c) about 80 mg/mL trehalose (e.g., α,α-trehalose dihydrate), (d) about 10 mM acetic acid-sodium acetate buffer, (e) about 4.5 mM calcium chloride, and (f) about 0.4 mg/mL polysorbate 80, and the pharmaceutical composition has a pH of about 5.0. In some embodiments of the pharmaceutical composition, the pharmaceutical composition comprises an intact anti-SOST antibody but does not comprise a broken anti-SOST antibody, or the pharmaceutical composition comprises an intact anti-SOST antibody and a broken anti-SOST antibody, and the broken anti-SOST antibody is a broken anti-SOST antibody resulting from HCDR3 breaking. In some embodiments, the broken anti-SOST antibody is a broken anti-SOST antibody resulting from the breaking at the amino acid residue at position 4 of the HCDR3 of an anti-SOST antibody. In some embodiments, the pharmaceutical composition comprises an intact anti-SOST antibody but does not comprise a broken anti-SOST antibody, wherein a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 27; or the pharmaceutical composition comprises an intact anti-SOST antibody and a broken anti-SOST antibody, wherein a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 27, and the proportion of the broken anti-SOST antibody to the total antibody amount is no more than 25% (e.g., no more than 0.1%, 0.2%, 0.3%, 0.4%, 0.5%, 1.0%, 1.5%, 2.0%, 3.0%, 4.0%, 4.5%, 5.0%, 6.0%, 7.0%, 8.0%, 9.0%, 9.5%, 10%, 11%, 12%, 13%, 14%, 15%, 16%, 17%, 18%, 19%, 20%, 21%, 22%, 23%, 24%, or 25%, or no more than any value between these point values; or the proportion of the broken anti-SOST antibody to the total antibody amount is any range between these point values; for example, the proportion of the broken anti-SOST antibody to the total antibody amount is 0.1% to 25%, 0.1% to 20%, 0.3% to 16%, 0.3% to 10%, 0.3% to 5%, 0.3% to 2%, 0.3% to 0.5%, or the like); in some embodiments, in the pharmaceutical composition, the proportion of the broken anti-SOST antibody with a heavy chain the amino acid sequence of which is set forth in SEQ ID NO: 27 to the total antibody amount is no more than 16%; in some embodiments, in the pharmaceutical composition, the proportion of the broken anti-SOST antibody with a heavy chain the amino acid sequence of which is set forth in SEQ ID NO: 27 to the total antibody amount is no more than 10%. In some embodiments, in the pharmaceutical composition, the proportion of the broken anti-SOST antibody with a heavy chain the amino acid sequence of which is set forth in SEQ ID NO: 27 to the total antibody amount is no more than 2.0%. In some embodiments, in the pharmaceutical composition, the proportion of the broken anti-SOST antibody with a heavy chain the amino acid sequence of which is set forth in SEQ ID NO: 27 to the total antibody amount is no more than 0.3%.

The disclosure also provides a pharmaceutical composition, wherein the pharmaceutical composition comprises an intact anti-SOST antibody and a broken anti-SOST antibody; a heavy chain of the intact anti-SOST antibody comprises the amino acid sequence set forth in SEQ ID NO: 22, and a light chain comprises the amino acid sequence set forth in SEQ ID NO: 25. In some embodiments, the broken anti-SOST antibody is a broken anti-SOST antibody resulting from HCDR3 breaking. In some embodiments, the broken anti-SOST antibody is a broken anti-SOST antibody resulting from the breaking at the amino acid residue at position 4 of the HCDR3 of an anti-SOST antibody.

In some embodiments of the pharmaceutical composition, the pharmaceutical composition comprises an intact anti-SOST antibody and a broken anti-SOST antibody, wherein a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 27, and the proportion of the broken anti-SOST antibody to the total antibody amount is no more than 0.1% to 25% (e.g., no more than 0.1%, 0.2%, 0.3%, 0.4%, 0.5%, 1.0%, 1.5%, 2.0%, 3.0%, 4.0%, 4.5%, 5.0%, 6.0%, 7.0%, 8.0%, 9.0%, 9.5%, 10%, 11%, 12%, 13%, 14%, 15%, 16%, 17%, 18%, 19%, 20%, 21%, 22%, 23%, 24%, or 25%, or no more than any value between these point values; or the proportion of the broken anti-SOST antibody to the total antibody amount is any range between these point values; for example, the proportion of the broken anti-SOST antibody to the total antibody amount is 0.1% to 25%, 0.1% to 20%, 0.3% to 16%, 0.3% to 10%, 0.3% to 5%, 0.3% to 2%, 0.3% to 0.5%, or the like); in some embodiments, in the pharmaceutical composition, the proportion of the broken anti-SOST antibody with a heavy chain the amino acid sequence of which is set forth in SEQ ID NO: 27 to the total antibody amount is no more than 16%; in some embodiments, in the pharmaceutical composition, the proportion of the broken anti-SOST antibody with a heavy chain the amino acid sequence of which is set forth in SEQ ID NO: 27 to the total antibody amount is no more than 10%. In some embodiments, in the pharmaceutical composition, the proportion of the broken anti-SOST antibody with a heavy chain the amino acid sequence of which is set forth in SEQ ID NO: 27 to the total antibody amount is no more than 2.0%. In some embodiments, in the pharmaceutical composition, the proportion of the broken anti-SOST antibody with a heavy chain the amino acid sequence of which is set forth in SEQ ID NO: 27 to the total antibody amount is no more than 0.3%.

In some embodiments of the pharmaceutical composition, the pharmaceutical composition comprises an intact anti-SOST antibody and a broken anti-SOST antibody, wherein a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 26, and the proportion of the broken anti-SOST antibody to the total antibody amount is no more than 4% (e.g., no more than 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.5%, or 4.0%, or no more than any value between these point values; or the proportion of the broken anti-SOST antibody to the total antibody amount is any range between these point values; for example, the proportion of the broken anti-SOST antibody to the total antibody amount is 0.1% to 4%, 0.1% to 2.8%, 0.1% to 2.5%, 0.1% to 1.5%, 0.4% to 1.4%, or the like); in some embodiments, in the pharmaceutical composition, the proportion of the broken anti-SOST antibody with a heavy chain the amino acid sequence of which is set forth in SEQ ID NO: 26 to the total antibody amount is no more than 2.3%; in some embodiments, in the pharmaceutical composition, the proportion of the broken anti-SOST antibody with a heavy chain the amino acid sequence of which is set forth in SEQ ID NO: 26 to the total antibody amount is no more than 1.4%. In some embodiments, in the pharmaceutical composition, the proportion of the broken anti-SOST antibody with a heavy chain the amino acid sequence of which is set forth in SEQ ID NO: 26 to the total antibody amount is no more than 0.7%. In some embodiments, in the pharmaceutical composition, the proportion of the broken anti-SOST antibody with a heavy chain the amino acid sequence of which is set forth in SEQ ID NO: 26 to the total antibody amount is no more than 0.4%.

In some embodiments of the pharmaceutical composition, the antibody is Ab-5; the pharmaceutical composition can prevent the heavy chain HCDR3 from breaking better than a pharmaceutical composition without an antioxidant (e.g., methionine). In some embodiments, the pharmaceutical composition is left to stand at a high temperature of 40 °C for 7 d or 30 d, or at 25 °C or 4 °C for 1, 3, 6, 12, 15 or 18 months, and the percent purity of intact antibody monomers is greater than or equal to 70% (e.g., greater than or equal to 80%, greater than or equal to 85%, greater than or equal to 90%, greater than or equal to 91%, greater than or equal to 92%, greater than or equal to 93%, greater than or equal to 94%, greater than or equal to 95%, greater than or equal to 96%, greater than or equal to 97%, greater than or equal to 98%, greater than or equal to 99% or 100%); in some embodiments, the pharmaceutical composition is left to stand at a high temperature of 40 °C for 30 d, and the purity of intact antibody monomers is greater than or equal to 70% (e.g., greater than or equal to 70%, e.g., greater than or equal to 75%, greater than or equal to 80%, greater than or equal to 85%, greater than or equal to 88%, greater than or equal to 89%, greater than or equal to 90%, greater than or equal to 91%, greater than or equal to 92%, greater than or equal to 93%, greater than or equal to 94%, greater than or equal to 95%, greater than or equal to 96%, greater than or equal to 97%, greater than or equal to 98%, greater than or equal to 99% or 100%). In some embodiments, the purity of the intact antibody monomer was measured by methods well known in the art, including but not limited to SEC size exclusion chromatography, NR-CE capillary gel electrophoresis, and the like.

In some embodiments, the disclosure also provides a lyophilized formulation, wherein the lyophilized formulation, upon reconstitution, can form the pharmaceutical composition according to one of the foregoing.

In some embodiments, the disclosure also provides a lyophilized formulation that is a lyophilized form of the pharmaceutical composition according to any of the foregoing. In some embodiments, the disclosure also provides a lyophilized formulation, wherein the formulation is obtained by lyophilizing the pharmaceutical composition according to any of the foregoing.

In some embodiments, the disclosure also provides a method for preparing a lyophilized formulation, comprising a step of lyophilizing the pharmaceutical composition according to any of the foregoing. In some embodiments, the lyophilizing comprises steps of pre-freezing, primary drying, and secondary drying in sequence. In some embodiments, the pre-freezing is from 5 °C to -40 °C to -50 °C, most preferably -45 °C, with no limitations on the degree of vacuum; for the primary drying, the temperature is -30 °C to 0 °C, most preferably -27 °C; the degree of vacuum is 0.05 mBar-0.2 mBar, most preferably 0.1 mBar; for the secondary drying, the temperature is 20 °C-30 °C, most preferably 25 °C, and the degree of vacuum is reduced from 0.05 mBar-0.2 mBar, most preferably 0.1 mBar, to 0.005 mBar-0.02 mBar, most preferably 0.01 mBar.

The disclosure also provides a lyophilized formulation prepared by the aforementioned method for preparing a lyophilized formulation.

In some embodiments, the disclosure also provides a reconstituted solution, wherein the reconstituted solution is prepared by reconstituting the lyophilized formulation according to any of the foregoing.

In some embodiments, the disclosure also provides a reconstituted solution, wherein the reconstituted solution is a reconstituted form of the lyophilized formulation according to any of the foregoing.

In some embodiments, the disclosure also provides a method for preparing the reconstituted solution according to any of the foregoing, comprising a step of reconstituting the aforementioned lyophilized formulation with a solution selected from the group consisting of, including but not limited to, water for injection, normal saline, or glucose solution. The reconstituting refers to dissolving the low-pressure lyophilized protein formulation in a diluent (selected from the group consisting of, including but not limited to, water for injection, normal saline, or glucose solution) so that the protein is dispersed in the reconstituted solution, thereby affording the corresponding reconstituted solution.

In some embodiments, the disclosure also provides a reconstituted solution obtained by the aforementioned method for preparing the reconstituted solution.

In some embodiments, the pharmaceutical composition or reconstituted solution according to any of the foregoing is a formulation for intravenous, subcutaneous, intraperitoneal, or intramuscular injection; in some embodiments, the pharmaceutical composition or reconstituted solution according to any of the forgoing is a formulation for intravenous injection. In some embodiments, the pharmaceutical composition or reconstituted solution according to any of the above is suitable for intravenous, subcutaneous, intraperitoneal, or intramuscular injection. In some embodiments, the pharmaceutical composition or reconstituted solution or lyophilized formulation according to any of the above is for use in the preparation of a medicament for intravenous, subcutaneous, intraperitoneal, or intramuscular injection.

In some embodiments, the disclosure also provides a product, comprising a container, wherein the container contains the pharmaceutical composition, lyophilized formulation, or reconstituted solution according to any of the foregoing. In some embodiments, the container is a tubular injection vial made of neutral borosilicate glass.

In some embodiments, the disclosure also provides a method for treating an SOST-related disease or condition, the method comprising administering to a subject in need thereof a therapeutically effective amount of: (A) the pharmaceutical composition according to any of the foregoing, (B) the lyophilized formulation according to any of the foregoing, (C) the reconstituted solution according to any of the foregoing, or (D) the product according to any of the foregoing.

In some embodiments, the disclosure also provides use of the pharmaceutical composition, lyophilized formulation, reconstituted solution, or product according to any of the foregoing in the preparation of a medicament for treating an SOST-related disease or condition.

In some embodiments, the disclosure also provides the pharmaceutical composition, lyophilized formulation, reconstituted solution, or product according to any of the foregoing for use in the treatment of an SOST-related disease or condition.

In some embodiments, the SOST-related disease or condition according to any of the foregoing is selected from the group consisting of: osteoporosis, osteopenia, osteoarthritis, rheumatoid arthritis, periodontal disease, and multiple myeloma; in some embodiments, the SOST-related disease or condition is osteoporosis.

### BRIEF DESCRIPTION OF THE DRAWINGS

FIG. 1: an NR-CE pattern for Ab-5 formulation 1;
FIG. 2: an R-CE pattern for Ab-5 formulation 1;
FIG. 3: a molecular weight pattern for fragment 1 of Ab-5 formulation 2;
FIG. 4: a molecular weight pattern for fragment 2 of Ab-5 formulation 2;
FIG. 5: a peptide map for Ab-5 formulation 2 after high-temperature degradation (standing at 40 °C for 30 d); and
FIG. 6: a peptide map for Ab-5 formulation 2 before high-temperature degradation.

### DETAILED DESCRIPTION

### Terminology

In order to facilitate the understanding of the disclosure, some technical and scientific terms are specifically defined below. Unless otherwise explicitly defined herein, all other technical and scientific terms used herein have the meanings generally understood by those of ordinary skill in the art to which the disclosure belongs.

"Antioxidant" refers to an agent that prevents or reduces the oxidation of an active pharmaceutical ingredient. Antioxidants include, but are not limited to: free radical scavengers (e.g., ascorbic acid, butylated hydroxytoluene (BHT), butylated hydroxyanisole (BHA), sodium sulfite, p-aminobenzoic acid, glutathione, propyl gallate, and the like), chelating agents (e.g., ethylenediaminetetraacetic acid (EDTA), diethylenetriaminepentaacetic acid (DTPA), nitrilotriacetic acid (NTA), N-2-acetamido-2-iminodiacetic acid (ADA), bis(aminoethyl)glycol ether, N,N,N',N'-tetraacetic acid (EGTA), trans-diaminocyclohexanetetraacetic acid (DCTA), amino acids (glutamic acid, aspartic acid, and the like), N-hydroxyethyliminodiacetic acid (HIMDA), N,N-bis-hydroxyethylglycine (bicine) and N-tris(hydroxymethyl)methylglycine (tricine), glycylglycine, sodium deoxycholate, ethylenediamine, propylenediamine, diethylenetriamine, triethylenetetraamine (trien), EDTA disodium salt, calcium EDTA oxalic acid, malate, citric acid, citric acid monohydrate and trisodium citrate dihydrate, and 8-hydroxyquinolinate), chain terminators (e.g., methionine and cysteine), and the like. In some embodiments, the antioxidant may be selected from the group consisting of: methionine, histidine, arginine, EDTA, citric acid, ascorbic acid, butylated hydroxytoluene, butylated hydroxyanisole, sodium sulfite, p-aminobenzoic acid, glutathione, propyl gallate, ethanol, benzyl alcohol, and the like. In some embodiments, the antioxidant is selected from the group consisting of methionine, histidine, and arginine. In some embodiments, the antioxidant is selected from methionine. In some embodiments, the antioxidant can effectively reduce the breaking at the HCDR3 of the antibody Ab-5. In some embodiments, the antioxidant can effectively prevent the heavy chain of the antibody Ab-5 from breaking at the amino acid residue at position 4 of the HCDR3.

"Buffer" refers to a buffering agent that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" refers to a buffer containing citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Viscosity modifier" refers to conventional pharmaceutical materials added to adjust the viscosity of a formulation. The viscosity modifier may be an inorganic salt and an amino acid salt; preferably, the inorganic salt is selected from the group consisting of sodium chloride, calcium chloride, magnesium chloride, or calcium acetate; preferably, the amino acid salt is selected from the group consisting of arginine hydrochloride, histidine hydrochloride, glycine hydrochloride, histidine acetate, and the like.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis, or reconstitution following centrifugation.

"Pharmaceutical composition" refers to a mixture containing one or more of the antibodies described herein (e.g., an anti-SOST antibody) or physiologically/pharmaceutically acceptable salts or pro-drugs thereof, and other chemical components, as well as other components such as physiologically/pharmaceutically acceptable carriers and excipients. The pharmaceutical composition is intended to promote administration to an organism and facilitate the absorption of the active ingredient so that it can exert its biological activity.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for aerosol or parenteral administration is phosphate buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Lyophilized formulation" refers to a formulation or a pharmaceutical composition obtained by lyophilizing a pharmaceutical composition or a formulation in liquid or solution form in vacuum. Generally, lyophilization includes pre-freezing, primary drying, and secondary drying. The purpose of pre-freezing is to freeze the product to obtain a crystalline solid; in some embodiments, the pre-freezing temperature is set at -45 °C, and the pre-freezing rate is set at 1 °C/min. Primary drying is the primary stage of lyophilizing a sample. The aim is to remove ice from the product while maintaining the shape of the product and minimizing damage to the product. If the temperature and the degree of vacuum are not properly selected during primary drying, it can lead to the collapse of the product. Higher temperatures and degrees of vacuum can both increase the lyophilization efficiency, but they also increase the risk of product collapse. In some embodiments, the temperature of the primary drying may be a temperature conventional in the art, for example, -30 °C-0 °C. Secondary drying, also known as desorption drying, is the main step of removing bound water from the product by creating an ultimate vacuum (0.01 mbar) and increasing the temperature (20-40 °C). As most biological products are sensitive to temperature, the secondary drying temperature is often set at a lower point of a temperature range, e.g., 25 °C. The lyophilization time is related to the freezer, the dose of the lyophilized formulation, and the container for the lyophilized formulation. Such time adjustments are well known to those skilled in the art.

Unless otherwise stated, the solvent in the pharmaceutical composition described herein in solution form is water.

As used herein, "pharmaceutical composition" and "formulation" are not mutually exclusive.

The pharmaceutical composition described herein can achieve a stabilizing effect: a pharmaceutical composition in which the antibody (e.g., an anti-SOST antibody) substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

In the disclosure, stable formulations include formulations in which no significant change is observed under the following conditions: stored at a refrigeration temperature (2-8 °C) for at least 3 months, preferably 6 months, more preferably 1 year, and even more preferably up to 2 years. In addition, stable liquid formulations further include liquid formulations that exhibit desirable features after storage at temperatures including 25 °C for periods including 1 month, 3 months, or 6 months. Typical examples for stability are as follows: generally, no more than about 10%, preferably no more than about 5%, of anti-SOST antibody aggregate or are degraded as measured by SEC-HPLC. The formulation is a pale yellow, nearly colorless and clear liquid, or a colorless and clear liquid, or is clear to slightly opalescent, by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed. In some embodiments, standing at a high temperature of 40 °C for one month, the anti-SOST antibody formulation of the disclosure exhibits an SEC monomer% of greater than or equal to 90% (e.g., greater than or equal to 90%, greater than or equal to 95%, greater than or equal to 96%, greater than or equal to 97%, greater than or equal to 98%, or greater than or equal to 99%) and/or a CE-SDS(NR)% of greater than or equal to 70% (e.g., greater than 70%, greater than 80%, greater than 90%, or greater than 95%).

An anti-SOST antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An anti-SOST antibody "retains its chemical stability" in a pharmaceutical formulation if it shows no significant chemical change. Chemical stability can be evaluated by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An anti-SOST antibody "retains its biological activity" in a pharmaceutical formulation if its biological activity at a given time is within a predetermined range of the biological activity exhibited during the preparation of the pharmaceutical formulation.

As used in the disclosure, the three-letter and single-letter codes for amino acids are described in J. Biol. Chem. 243, p3558 (1968).

As used in the disclosure, the singular forms "a", "an", and "the" include plural referents unless the context clearly dictates otherwise.

Unless the context clearly dictates otherwise, throughout the description and the claims, the words "comprise", "have", "include", and the like, should be construed in an inclusive sense as opposed to an exclusive or exhaustive sense.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

"Optional" or "optionally" means that the event or circumstance subsequently described may, but does not necessarily occur; this description includes instances where the event or circumstance occurs or does not occur. For example, "optionally comprising a heavy chain variable region of SEQ ID NO: 11" means that the antibody heavy chain variable region of the particular sequence may, but does not necessarily, exist.

As used herein, the term "about" or "approximately" means that a value is within an acceptable error range for the particular value determined by one of ordinary skill in the art; the value depends on how it is measured or determined (i.e., the limitations of the measurement system). Herein, unless otherwise specified (e.g., a pH range), the term "about" may indicate being within a range of ±10% of the particular value that follows, optionally being within a range of ±10%, ±9%, ±8%, ±7%, ±6%, or ±5% of the particular value that follows.

While the disclosure provides content ranges or values, those of ordinary skill in the art will understand that the content ranges or values encompass acceptable error ranges for the specific value determined.

The term "sclerostin" or "SOST" or "SOST protein" refers to the sclerostin (or SOST) gene expression product (protein). Unless otherwise specified, e.g., murine SOST (m-SOST) or cynomolgus monkey SOST (cyno-SOST), the term refers to human SOST (h-SOST). As used in the disclosure, human, murine, and cynomolgus monkey SOSTs have nucleotide sequences available from GenBank; for example, NP_079513.1 provides a human SOST protein sequence.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W. The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies and antigen-binding fragments (or antigen-binding moieties) so long as they exhibit the desired antigen-binding activity. "Natural antibody" refers to a naturally-occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as a variable heavy domain or heavy chain variable region, followed by a heavy chain constant region. An IgG heavy chain constant region (CH) generally comprises three constant domains (CH1, CH2, and CH3). Similarly, from the N-terminus to the C-terminus, each light chain has one variable region (VL), also known as a variable light domain or light chain variable domain, followed by one constant light domain (light chain constant region, CL). The term "full-length antibody", "intact antibody", and "whole antibody" are used herein interchangeably and refer to an antibody having a substantially similar structure to a native antibody structure or having heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are exemplary, as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise indicated, the "Kabat" numbering scheme is applied to the sequences of the variable regions and CDRs in examples of the disclosure.

The term "broken antibody" refers to a molecule that differs from an intact antibody-it is part of an intact antibody. In the disclosure, in some embodiments, the broken anti-SOST antibody is a broken antibody resulting from the breaking at the HCDR3 of an antibody; in some embodiments, the broken anti-SOST antibody is a broken antibody resulting from the breaking at the amino acid residue at position 4 of the HCDR3 of Ab-5; in some embodiments, the broken antibody is a broken anti-SOST antibody with a heavy chain the amino acid sequence of which is set forth in SEQ ID NO: 26; in some embodiments, the broken antibody is a broken anti-SOST antibody with a heavy chain the amino acid sequence of which is set forth in SEQ ID NO: 27; as the terminal K of the heavy chain sequence of the humanized antibody Ab-5 may be removed during the antibody expression process, the heavy chain of the broken anti-SOST antibody may or may not have a K at the C-terminus.

In the disclosure, unless otherwise indicated, the proportion of a broken anti-SOST antibody to the total antibody amount is characterized by the non-reduced CE purity of the broken anti-SOST antibody: the proportion of a broken anti-SOST antibody to the total antibody amount = peak of the broken anti-SOST antibody/peaks of all antibodies × 100% (where the peak of the broken anti-SOST antibody refers to the area of the peak of the broken anti-SOST antibody in the sample, and the peaks of all antibodies refers to the combined area of the peaks of all antibodies in the sample). In some embodiments, in the pharmaceutical composition, the proportion of the broken anti-SOST antibody to the total antibody amount is the proportion of the broken anti-SOST antibody to the total antibody amount after the pharmaceutical composition is left to stand at a high temperature of 40 °C for 7 d or 30 d, or the proportion of the anti-SOST antibody to the total antibody amount after the pharmaceutical composition is left to stand at 25 °C or 4 °C for 1, 3, 6, 12, or 18 months, or the proportion of the broken anti-SOST antibody to the total antibody amount after the pharmaceutical composition is left to stand under other conditions.

The term "antigen-binding fragment" refers to a molecule that differs from an intact antibody; it comprises a moiety of an intact antibody that binds to an antigen to which the intact antibody binds. Examples of antigen-binding fragments include, but are not limited to, Fv, Fab, Fab', Fab'-SH, F(ab')2, single-domain antibodies, single-chain Fab (scFab), diabodies, linear antibodies, single-chain antibody molecules (e.g., scFv), and multispecific antibodies formed from antigen-binding fragments. In some embodiments, the antigen-binding fragment of the antibody is a monovalent Fab (i.e., Fab), a divalent Fab (F(ab)₂), a trivalent Fab fragment (F(ab)₃), a multivalent Fab (two or more Fabs), or may be a monospecific, bispecific, or multispecific antigen-binding fragment comprising at least one Fab fragment.

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the disclosure is a monoclonal antibody. The antibody of the disclosure may be an antibody derived from an animal (e.g., an antibody derived from a murine, fowl, rabbit, camel, monkey, or the like), a chimeric antibody, or a humanized antibody.

The term "chimeric" antibody refers to an antibody in which a portion of the heavy and/or light chain in the antibody is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1: 1 interaction between members of a binding pair (e.g., an antibody and an antigen). The affinity of a molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET). In some embodiments, the KD value is determined by Biacore.

The term "antigen" refers to a molecule or portion of a molecule that is capable of being bound by a selective binding agent, such as an antigen-binding protein (e.g., an antibody), and that is also capable of being used in an animal to produce an antibody that is capable of binding to the antigen. An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody (e.g., an anti-SOST antibody) is capable of binding to a certain antigen or an epitope in the antigen with higher affinity than to other antigens or epitopes. Generally, an antibody binds to an antigen or an epitope in the antigen with an equilibrium dissociation constant (KD) of about 1 × 10⁻⁷ M or less (e.g., less than 10 nM, 3 nM, 1 nM, 0.3 nM, 0.1 nM or less). In some embodiments, the KD for the binding of an antibody to an antigen is 10% or less (e.g., 1%) of the KD for the binding of the antibody to a nonspecific antigen (e.g., BSA or casein). KD may be determined using known methods, for example, by a BIACORE^{®} surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term sequence "identity" refers to the degree (percentage) to which the amino acids/nucleic acids of two sequences are identical at equivalent positions when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

"Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting the reagent with the cells and contacting the reagent with fluid, where the fluid is in contact with the cells. "Administering", "giving", and "treating" also refer to treating, e.g., cells by reagents, diagnosis, binding compositions or by another cell *in vitro* and *ex vivo.* "Treating", when applied to humans, veterinary, or research subjects, refers to therapeutic treatment, preventive or prophylactic measures, and research and diagnostic applications.

"Treatment" refers to administering, either internally or externally, a therapeutic agent, for example, comprising any of the pharmaceutical compositions of the disclosure, to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the disclosure (for example, treatment methods or products) may not be effective in alleviating the symptoms of each disease of interest, they shall reduce the symptoms of the disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Effective amount" comprises an amount sufficient to ameliorate or prevent a symptom or sign of a medical condition. An effective amount also refers to an amount sufficient to allow or facilitate diagnosis. The effective amount for a subject may vary depending on factors such as the condition to be treated, the general health of the subject, the method and dose of administration, and the severity of side effects. An effective amount may be the maximum dose or administration regimen to avoid significant side effects or toxic effects. The subject of the disclosure may be an animal or a human subject.

The pharmaceutical composition of the disclosure may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection. Various administration schedules are contemplated herein, including but not limited to, single administration or multiple administrations at multiple time points, bolus administration, and pulse infusion. In some embodiments, the pharmaceutical composition of the disclosure is administered intravenously.

The pharmaceutical composition of the disclosure will be formulated, administered, and applied in a manner consistent with Good Medical Practice. Factors considered in this context include the specific condition being treated, the specific mammal being treated, the clinical state of the individual patient, the cause of the condition, the site of delivery of the agent, the method of administration, the timing of administration, and other factors known to medical practitioners. Optionally, the pharmaceutical composition may also be formulated with one or more additional agents used for preventing or treating the condition. The effective amount of such additional agents depends on the amount of the antigen-binding molecule present in the pharmaceutical composition, the type of the condition or treatment, and other factors. Such additional agents may be used in the same dosages and with routes of administration as described herein, or in about 1 to 99% of the dosages described herein, or in any dosage and by any route that is empirically/clinically determined to be appropriate.

The disclosure is further described below with reference to examples or test examples; however, these examples or test examples are not intended to limit the scope of the disclosure. Experimental procedures without specific conditions indicated in the examples or test examples are generally conducted according to conventional conditions, or according to conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

The method for preparing the SOST antibodies in the application is described in the patent application WO2016145961A1, the disclosure of which is incorporated herein by reference in its entirety.

### Example 1: Production of Anti-Human SOST Monoclonal Antibody

An anti-human SOST monoclonal antibody was produced by immunization of mice. Laboratory SJL white mice, female, 6 weeks of age (Beijing Vital River Laboratory Animal Technology Co., Ltd., animal production license number: SCXK(Beijing)2012-0001). Housing environment: SPF. The purchased mice were housed in a laboratory environment for 1 week, with a 12/12 hour light/dark cycle, at a temperature of 20-25 °C with humidity at 40-60%. Acclimated mice were divided into 2 groups (A/B) of 10. The antigen for immunization was a His-tagged SOST recombinant protein (His-h-SOST). Group A was emulsified with Freund's adjuvant (sigma Lot Num: F5881/F5506): the first immunization was performed with complete Freund's adjuvant (CFA), and the subsequent boost immunizations were performed with incomplete Freund's adjuvant (IFA). The antigen-to-adjuvant ratio was 1:1, 200 µL/200 µg/mouse (first immunization), 200 µL/100 µg/mouse (boost immunization). Group B was cross-immunized with Titermax (sigma Lot Num: T2684) and Alum (Thremo Lot Num: 77161). The antigen-to-adjuvant (titermax) ratio was 1:1, and the antigen-to-adjuvant (Alum) ratio was 3:1, 200 µL/200 µg/mouse (first immunization), 200 µL/100 µg/mouse (boost immunization). The antigen was emulsified before inoculation, and inoculation was performed at days 0, 14, 28, 42, and 56.

At day 0, each mouse in group A/B was intraperitoneally (IP) injected with 50 µg of the emulsified antigen. At day 14, each mouse was administered a subcutaneous (sc) multisite (typically 6-8 sites on the back) injection of 25 µg. At days 28 and 42, dorsal or intraperitoneal injections of the antigen were selected based on dorsal lumping and abdominal swelling. Boost immunization was performed 3 days before splenocyte fusion, and each mouse was intraperitoneally (IP) injected with 200 µg of antigen solution in normal saline.

Blood tests were performed at days 22, 36, 50, and 64, and mouse serum was assayed by ELISA for binding activity with human sclerostin. After the 4th immunization, mice with high serum antibody titers that tended to plateau were selected for splenocyte fusion. An optimized PEG-mediated fusion procedure was used to fuse spleen lymphocytes with the myeloma Sp2/0 cells (ATCC^{®} CRL-8287^{™}) to obtain hybridoma cells. Mouse serum was assayed for the activity of the anti-SOST antibody in blocking the binding of human SOST to human LRP-6, and the monoclonal hybridoma cell strain Ab-1, which showed good *in vitro* activity, was selected. The activity results are shown in Table 2.

**Table 2. The in vitro activity of the murine antibody**

| Candidate antibody | EC₅₀ (nM) | IC₅₀ (nM) |
|---|---|---|
| Ab-1 | 0.701 | 9.91 |

### Example 2: Humanization of Murine Anti-Human Sclerostin Antibody

The monoclonal hybridoma cell strain Ab-1, which showed good *in vitro* activity, was selected, and its monoclonal antibody sequence was cloned, followed by humanization, recombinant expression, and activity evaluation.

The process of cloning the sequence from the hybridoma is as follows: hybridoma cells in the logarithmic growth phase were collected, and RNA was extracted with Trizol (Invitrogen, 15596-018) (by following the instructions in the kit) and reverse-transcribed (PrimeScript^{™} Reverse Transcriptase, Takara, cat # 2680A); the cDNA obtained from reverse transcription was amplified by PCR using mouse Ig-Primer Set (Novagen, TB326 Rev.B 0503) and then sent to a sequencing company for analysis, and the amino acid sequences of the variable regions of the hybridoma cell strain Ab-1 were obtained:
The heavy chain variable region of the hybridoma cell Ab-1 (SEQ ID NO: 1), 
EVQLQQSGPELVKPGTSVKIPCQTSGYTFTDYNLDWLKQRPGESLEWIGDIDPN
NGDILYNQKFRDKATLTVDTSSNTAYLELRSLTSEDTAVYYCARRWAYYFDYWG
QGTTLTISS
The light chain variable region of the hybridoma cell Ab-1 (SEQ ID NO: 2),

NIVMTQTPKLLFVSAGDRITITCKASQSVSNDVAWYQQKPGQSPKLLIYYTSNRF
TGVPDRFTGSGYGTDFTLTINTVQAEDLAVYFCQQDYSSPVTFGAGTKLELK

Methods for humanizing murine anti-human sclerostin monoclonal antibodies are well known in the art. In sum, a human constant domain is used to replace the parent (murine antibody) constant domain, and based on the homology between the murine and human antibodies, a human germline template with high homology is selected for humanization. The method is specifically as follows:

### 1. The CDR regions of the murine anti-sclerostin antibody

The amino acid residues of the CDRs of the antibody VH/VL were determined and annotated by the Kabat numbering system. The CDR sequences of the murine antibody Ab-1 are described in Table 3:

**Table 3. The CDR sequences of the antibody Ab-1**

| | |
|---|---|
| Heavy chain CDR1 | DYNLD (SEQ ID NO: 3) |
| Heavy chain CDR2 | DIDPNNGDILYNQKFRD (SEQ ID NO: 4) |
| Heavy chain CDR3 | RWAYYFDY (SEQ ID NO: 5) |
| Light chain CDR1 | KASQSVSNDVA (SEQ ID NO: 6) |
| Light chain CDR2 | YTSNRFT (SEQ ID NO: 7) |
| Light chain CDR3 | QQDYSSPVT (SEQ ID NO: 8) |

### 2. Selection of human germline FR region sequences

Based on the typical CDR structure of the VH/VL of the murine antibody obtained, the heavy and light chain variable region sequences were compared with an antibody GermLine database to obtain a human germline template with high homology. The human germline light chain framework regions were from the human κ light chain gene. In the disclosure, the human germline light chain template IGKV1-39*01 or IGKV4-1*01 was preferred. The human germline heavy chain framework regions were from human heavy chains. In the disclosure, the human germline heavy chain template IGHV1-18*01 was preferred. The CDR regions of the murine antibody Ab-1 were grafted onto the selected humanization template to replace the humanized variable regions, followed by recombination with a human constant region. Then, based on the three-dimensional structure of the murine antibody, embedded residues, residues that directly interact with the CDR regions, and residues that have a major influence on the conformation of the VL and VH were subjected to back mutation. In some embodiments, chemically unstable amino acid residues in the CDR regions were optimized, and the heavy chain HCDR2 was optimized to obtain a new heavy chain HCDR2 sequence: DIDPNDGDILYNQKFRD (SEQ ID NO: 9). The heavy chain variable regions of the obtained humanized antibodies are set forth in SEQ ID NOs: 10-12; they were fused with any heavy chain constant region selected from the group consisting of SEQ ID NOs: 16-18 to construct humanized antibody heavy chains. The light chain variable regions are set forth in SEQ ID NOs: 13-15; they were fused with a light chain constant region set forth in SEQ ID NO: 19 to construct humanized antibody light chains.
1) The heavy chain variable regions of humanized antibodies:
   The heavy chain variable region of Ab-5 (SEQ ID NO: 10), 
EVQLVQSGAEVKKPGASVKVSCKASGYTFTDYNLDWLRQAPGEGLEWIGDIDP
NDGDILYNQKFRDRVTMTTDTSTSTAYMELRSLRSDDTAVYYCARRWAYYFDY
WGQGTTVTVSS
   The heavy chain variable region of Ab-9 (SEQ ID NO: 11), 
EVQLVQSGAEVKKPGASVKVSCKASGYTFTDYNLDWLRQAPGEGLEWIGDIDP
NNGDILYNQKFRDRVTMTTDTSTSTAYMELRSLRSDDTAVYYCARRWAYYFDY
WGQGTTVTVSS
   The heavy chain variable region of Ab-10 (SEQ ID NO: 12), 
EVQLVQSGAEVKKPGASVKVSCKASGYTFTDYNLDWVRQAPGQGLEWMGDI
DPNNGDILYNQKFRDRVTMTTDTSTSTAYMELRSLRSDDTAVYYCARRWAYYF
DYWGQGTTVTVSS
2) The heavy chain constant regions of humanized antibodies are optionally selected from the group consisting of the following sequences:
   The heavy chain constant region of human IgG4 with K deleted from the C-terminus (with K deleted from the C-terminus) (SEQ ID NO: 16): 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
   The heavy chain constant region of human IgG4 (SEQ ID NO: 17): 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
   The heavy chain constant region of human IgG2 (SEQ ID NO: 18):
3) The light chain variable regions:
   The light chain variable region of Ab-5 (SEQ ID NO: 13): 
DIVMTQSPSSLSASVGDRVTITCKASQSVSNDVAWYQQKPGKSPKLLIYYTSNRF
TGVPDRFSGSGSGTDFTLTISSLQPEDFATYFCQQDYSSPVTFGGGTKVEIK
   The light chain variable region of Ab-9 (SEQ ID NO: 14): 
NIVMTQSPSSLSASVGDRVTITCKASQSVSNDVAWYQQKPGKSPKLLIYYTSNRF
TGVPDRFSGSGSGTDFTLTISSLQPEDFATYFCQQDYSSPVTFGGGTKVEIK
   The light chain variable region of Ab-10 (SEQ ID NO: 15): 
DIQMTQSPSSLSASVGDRVTITCKASQSVSNDVAWYQQKPGKAPKLLIYYTSNR
FTGVPSRFSGSGSGTDFTLTISSLQPEDFATYYCQQDYSSPVTFGGGTKVEIK
4) The light chain κ constant region (SEQ ID NO: 19):

The antibodies above were cloned, expressed, and purified using gene cloning and recombinant expression methods. Through methods such as binding ELISA, antigen-receptor binding blocking experiments, Biacore, and cell viability assays, the humanized antibodies Ab-5, Ab-9, and Ab-10, which retained activity best, were finally selected. Their sequences are as follows:
The amino acid sequence of the heavy chain of Ab-10 (SEQ ID NO: 20): 
EVQLVQSGAEVKKPGASVKVSCKASGYTFTDYNLDWVRQAPGQGLEWMGDI
DPNNGDILYNQKFRDRVTMTTDTSTSTAYMELRSLRSDDTAVYYCARRWAYYF
DYWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
The amino acid sequence of the light chain of Ab-10 (SEQ ID NO: 23): 
DIQMTQSPSSLSASVGDRVTITCKASQSVSNDVAWYQQKPGKAPKLLIYYTSNR
FTGVPSRFSGSGSGTDFTLTISSLQPEDFATYYCQQDYSSPVTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
The amino acid sequence of the heavy chain of Ab-9 (SEQ ID NO: 21): 
EVQLVQSGAEVKKPGASVKVSCKASGYTFTDYNLDWLRQAPGEGLEWIGDIDP
NNGDILYNQKFRDRVTMTTDTSTSTAYMELRSLRSDDTAVYYCARRWAYYFDY
WGQGTTVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK
The amino acid sequence of the light chain of Ab-9 (SEQ ID NO: 24)

NIVMTQSPSSLSASVGDRVTITCKASQSVSNDVAWYQQKPGKSPKLLIYYTSNRF
TGVPDRFSGSGSGTDFTLTISSLQPEDFATYFCQQDYSSPVTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

The amino acid sequence of the heavy chain of Ab-5 (SEQ ID NO: 22):

EVQLVQSGAEVKKPGASVKVSCKASGYTFTDYNLDWLRQAPGEGLEWIGDIDP
NDGDILYNQKFRDRVTMTTDTSTSTAYMELRSLRSDDTAVYYCARRWAYYFDY
WGQGTTVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLG(K)

Notes: As the terminal K of the heavy chain sequence of the humanized antibody Ab-5 may be removed during the antibody expression process, the final product's heavy chain may or may not have a terminal K. However, this does not affect the performance of the product itself.

The amino acid sequence of the light chain of Ab-5 (SEQ ID NO: 25):
DIVMTQSPSSLSASVGDRVTITCKASQSVSNDVAWYQQKPGKSPKLLIYYTSNRF TGVPDRFSGSGSGTDFTLTISSLQPEDFATYFCQQDYSSPVTFGGGTKVEIKRTVA APSVFIFPPSDEQEKSGTASVVCEENNFYPREAKVQWKVDNAEQSGNSQESVTE QDSKDSTYSLSSTLTLSKADYEKHKVYACEVTHQGLSSPVTKSFNRGEC The amino acid sequences of the antibody fragments resulting from the breaking at the amino acid residue at position 4 of the HCDR3 region of the heavy chain of Ab-5 are as follows:
The amino acid sequence of antibody fragment H1 (SEQ ID NO: 26): 
EVQLVQSGAEVKKPGASVKVSCKASGYTFTDYNLDWLRQAPGEGLEWIGDIDP
NDGDILYNQKFRDRVTMTTDTSTSTAYMELRSLRSDDTAVYYCARRWA
The amino acid sequence of antibody fragment H2 (SEQ ID NO: 27):

YFDYWGQGTTVTVSSASTKGPSVFPLAPCSRSTSESTAALGCLVKDYFPEPV
TVSWNSGALTSGVHTFPAVLQSSGLYSLSSVVTVPSSSLGTKTYTCNVDHKPSNT
KVDKRVESKYGPPCPPCPAPEFLGGPSVFLFPPKPKDTLMISRTPEVTCVVVDVS
QEDPEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVM
HEALHNHYTQKSLSLSLG

### Example 3: Anti-SOST Antibody Formulations

The equipment, antibodies, and methods used in the preparation and analysis of formulations are shown below.

### SEC molecular exclusion chromatography:

This is a method for analyzing the separation of asoluteby the relative relationship between the pore size of the gel pores and the size of the polymer sample molecule coil. SEC% (SEC monomer content percentage) = A monomer / A total × 100% (A monomer represents the peak area of the main peak monomer in the sample, and A total represents the sum of all peak areas).

Instrument for SEC analysis: Agilent HPLC 1260; column: waters, XBridge BEH200Å SEC (7.8 × 300 mm 3.5 µm)

### NR-CE (also known as CE-SDS (NR)) capillary gel electrophoresis:

This is a method of moving the gel into a capillary as a supporting medium for electrophoresis and performing separation according to the molecular weight of the sample under a certain voltage.

Non-reduced CE purity percentage = A main peak/A total × 100% (A main peak is the peak area of the main peak in the sample, and A total is the sum of all peak areas). Instrument for CE analysis: Beckman capillary electrophoresis apparatus, model: plus800.

### R-CE (also known as Reduced-CE, CE-SDS(R), or CE) capillary gel electrophoresis:

This is a method of moving the gel into a capillary as a supporting medium for electrophoresis and performing separation according to the molecular weight of the sample under a certain voltage.

Instrument for R-CE analysis: Beckman capillary electrophoresis apparatus, model: plus800.

### iCIEF imaged capillary isoelectric focusing electrophoresis:

This is a technique for separation according to the difference of isoelectric points (pI) of proteins.

iCIEF neutral peak content percentage = neutral peak area/total area × 100% (total area represents the sum of areas of acidic, neutral, and basic peaks).

Instrument for iCIEF analysis: simple protein, model: muarice.

### Osmotic pressure determination:

The freezing point method is used for determining the osmotic pressure. On the basis of the directly proportional relation between the freezing point depression value and the molar concentration of the solution, the freezing point of a solution is determined by using a high-sensitivity temperature-sensing element, and then converted into the osmotic pressure through electric quantity.

Instrument for osmotic pressure determination: Loser, model: OM815.

### Protein concentration determination:

The anti-SOST antibody used in the following formulation examples was Ab-5. The concentration of the antibody was calculated on a protein concentration basis.

Instrument for protein concentration determination: ultraviolet-visible spectrophotometer; model: Nano Drop oneC; optical path length: 1 mm.

### Example 4: Identification of Ab-5 Antibody Fragments

In a 10 mM pH 5.0 acetic acid-sodium acetate buffer system, an anti-SOST antibody (Ab-5) solution formulation 1 was prepared with a protein concentration of 100 mg/mL, a calcium chloride concentration of 4.5 mM, an α,α-trehalose dihydrate concentration of 80 mg/mL, and a polysorbate 80 concentration of 0.4 mg/mL.

In addition, in a 10 mM pH 5.0 acetic acid-sodium acetate buffer system, an anti-SOST antibody (Ab-5) solution formulation 2 was prepared with a protein concentration of 50 mg/mL, an α,α-trehalose dihydrate concentration of 80 mg/mL, and a polysorbate 80 concentration of 0.4 mg/mL.

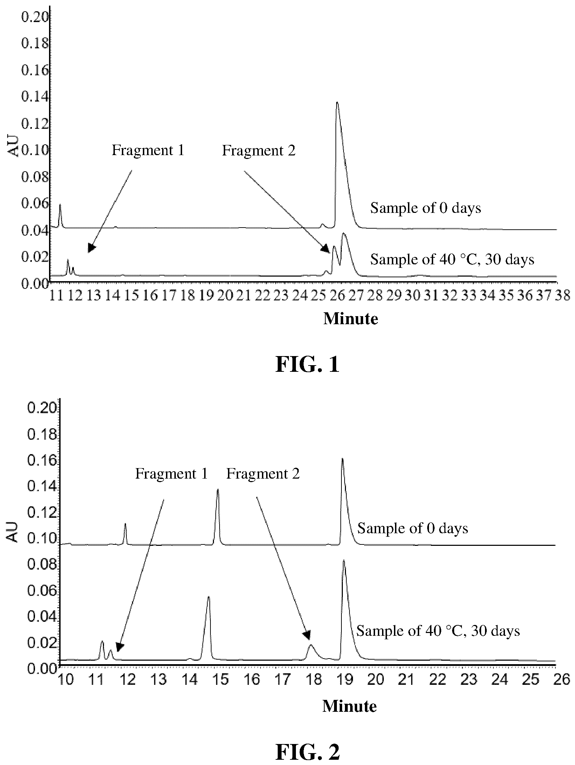

Formulation 1 was added to 2 mL vials at 1.0 mL/vial, and the vials were sealed with film-coated rubber stoppers and left to stand at 40 °C for 30 d. Antibody fragments in the formulation were preliminarily identified by NR-CE and R-CE. The experimental results are shown in FIG. 1 and FIG. 2.

The experimental results show that after standing at 40 °C for 30 d, formulation 1 exhibited the formation of fragment 1 and fragment 2 compared to formulation 1 on day 0; the NR-CE pattern shows that the molecular weight of fragment 1 was slightly greater than 10 kDa; the R-CE pattern shows that fragment 2 occurred between the light and heavy chains, suggesting that the fragments resulted from the breaking of the antibody heavy chain.

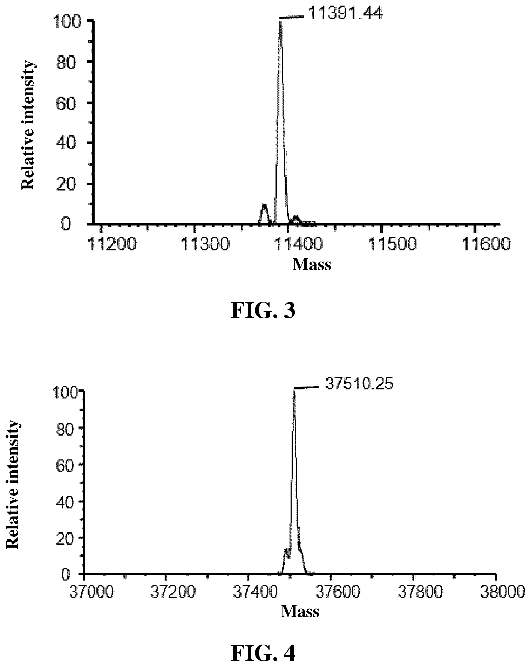

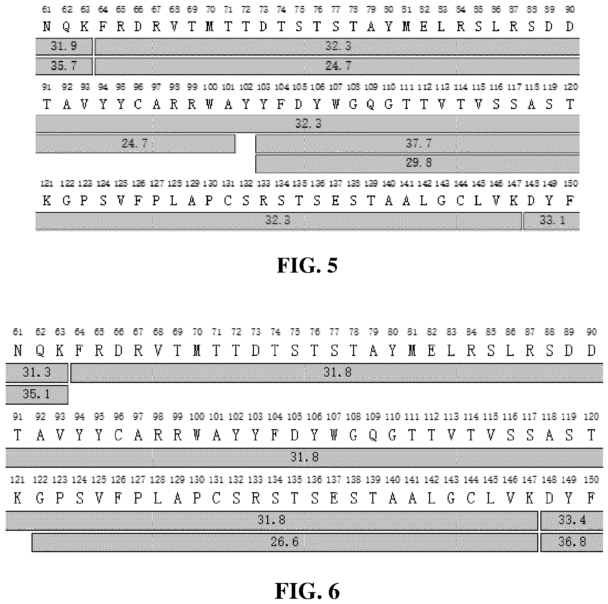

In addition, formulation 2 was added to 2 mL vials at 1.0 mL/vial, and the vials were sealed with film-coated rubber stoppers and left to stand at 40 °C for 30 d. Molecular weight after deglycosylation and reduction and peptide maps were used to further determine whether fragment 1 and fragment 2 resulted from the breaking of the antibody heavy chain. The experimental results are shown in FIG. 3, FIG. 4, FIG. 5, and FIG. 6. The experimental results for molecular weight after deglycosylation and reduction show that the molecular weight of fragment 1 was 11.39144 kDa, and the molecular weight of fragment 2 was 37.51025 kDa. These results agree with those described above for formulation 1. In addition, the peptide mapping results show that after degradation at a high temperature of 40 °C, formulation 2 exhibited the formation of peptide fragment 1 (a polypeptide formed from the amino acid residues at positions 1 to 101 of the heavy chain of Ab-5 (the amino acid sequence is set forth in SEQ ID NO: 26), hereinafter referred to as antibody fragment H1) and peptide fragment 2 (a polypeptide formed from the amino acid residues at positions 103 to 443 of the heavy chain of Ab-5 (the amino acid sequence is set forth in SEQ ID NO: 27), hereinafter referred to as antibody fragment H2), which did not result from enzymatic cleavage, compared to formulation 2 on day 0, suggesting that the amino acid residue at position 4 of the HCDR3 of the heavy chain of the antibody Ab-5 (amino acid residue position 102 (natural sequence numbering) of the heavy chain) is a easily breakable site. The theoretical molecular weight of antibody fragment H1 was calculated at 11.39356 kDa, and the theoretical molecular weight of antibody fragment H2 was calculated at 37.50691 kDa. These results agree with the molecular weight of fragment 1 and fragment 2 in FIG. 3 and FIG. 4.

In summary, the antibody Ab-5 easily breaks at around the amino acid residue at position 4 of the heavy chain HCDR3, producing antibody fragments.

### Example 5: Inhibition of Breaking of HCDR3 of Antibody Ab-5 by Antioxidants

In a 10 mM pH 5.0 acetic acid-sodium acetate buffer system, anti-SOST antibody (Ab-5) solutions were prepared with a protein concentration of 100 mg/mL, a calcium chloride concentration of 4.5 mM, an α,α-trehalose dihydrate concentration of 80 mg/mL (72 mg/mL on an anhydrous trehalose basis), and a polysorbate 80 concentration of 0.4 mg/mL, each containing one of the following antioxidants:
(1) N/A (no antioxidant was added)
(2) 50 mM histidine
(3) 50 mM methionine
(4) 50 mM arginine

The formulations were added to 2 mL vials at 1.0 mL/vial, and the vials were sealed with film-coated chlorobutyl rubber stoppers and then left to stand at a high temperature (40 °C). The stability of the formulations was evaluated based on their appearance and NR-CE results.

The experimental results are shown in Table 4. The appearance results show that after standing at a high temperature of 40 °C for 30 d, the arginine group was slightly opalescent, and the other groups were all clear and transparent. The NR-CE results show that the antibody purity NR-CE percentage of the group with methionine, histidine, or arginine was significantly superior to that of the group without the antioxidant, suggesting a significant reduction in the number of antibody fragments resulting from the breaking at the amino acid residue at position 4 of the HCDR3 of Ab-5. Antioxidants such as methionine can inhibit the breaking at the amino acid residue at position 4 of the HCDR3 of the antibody Ab-5.

**Table 4. The stability results for Ab-5 formulations containing different antioxidants**

| Antioxidant type | Time | Appearance | NR-CE% | | | | | |
|---|---|---|---|---|---|---|---|---|
| | | | Ab-5 main peak | Antibody fragment H1 | Antibody fragment H2 | Antibody fragments H1 + H2 | Other fragments | Aggregate |
| None | 0 | Clear | 96.2 | NA | 0.5 | 0.5 | 3.2 | 0.1 |
| | 30 d | Clear | 59.6 | 4.4 | 26.0 | 30.4 | 7.7 | 2.3 |
| 50 mM histidine | 0 | Clear | 96.5 | 0.0 | 0.0 | 0.0 | 3.3 | 0.2 |
| | 30 d | Clear | 88 | 0.6 | 4.6 | 5.2 | 6 | 0.8 |
| 50 mM methionine | 0 | Clear | 96.8 | 0.0 | 0.0 | 0 | 3.1 | 0.1 |
| | 30 d | Clear | 91.9 | 0.4 | 1.9 | 2.3 | 5 | 0.8 |
| 50 mM arginine | 0 | Clear | 96.5 | 0.0 | 0.0 | 0.0 | 3.4 | 0.1 |
| | 30 d | Slightly opalescent | 71.1 | 2.8 | 18.1 | 20.9 | 6.6 | 1.4 |

| | | | | | | | | |
|---|---|---|---|---|---|---|---|---|
| Notes: In the table, "antibody fragments H1 + H2" refers to the NR-CE% for the peaks of antibody fragment H1 (the amino acid sequence is set forth in SEQ ID NO: 26) and antibody fragment H2 (the amino acid sequence is set forth in SEQ ID NO: 27) resulting from the breaking at the amino acid residue at position 4 of the HCDR3 of Ab-5; "other fragments" refers to Ab-5 antibody fragments other than antibody fragment H1 and antibody fragment H1; "NA" means that the content was too low to be determined. | | | | | | | | |

### Example 6: Screening for Antioxidant Concentration

In a 10 mM pH 5.0 acetic acid-sodium acetate buffer system, anti-SOST antibody (Ab-5) solutions were prepared with a protein concentration of 100 mg/mL, a calcium chloride concentration of 4.5 mM, an α,α-trehalose dihydrate concentration of 80 mg/mL, and a polysorbate 80 concentration of 0.4 mg/mL, containing 5 mM, 10 mM, and 25 mM methionine.

The formulations were added to 2 mL vials at 1.0 mL/vial, and the vials were sealed with film-coated chlorobutyl rubber stoppers and then left to stand at a high temperature (40 °C). The stability of the formulations was evaluated based on their appearance and NR-CE results.

The experimental results are shown in Table 5. The experimental results show that the addition of methionine can significantly improve the stability of the samples, and the stability of the formulations increased somewhat as the amount of methionine increased. After standing at a high temperature of 40 °C for 30 d, the formulation with 10 mM methionine still had an Ab-5 purity NR-CE percentage of up to 85%.

**Table 5. The stability results for Ab-5 formulations containing different concentrations of methionine**

| Methionine concentration (mM) | Time | NR-CE% | | | | Appearance |
|---|---|---|---|---|---|---|
| | | Ab-5 main peak | Antibody fragment H1 | Antibody fragment H2 | Antibody fragments H1 + H2 | |
| 5 | 0 | 97.7 | 0.0 | 0.0 | 0.0 | Clear |
| | 30 d | 77.9 | 2.3 | 15.9 | 18.2 | Clear |
| 10 | 0 | 97.5 | 0.0 | 0.0 | 0.0 | Clear |
| | 30 d | 85 | 1.4 | 9.7 | 11.1 | Clear |
| 25 | 0 | 97.8 | 0.0 | 0.0 | 0.0 | Clear |
| | 30 d | 90.7 | 0.7 | 4.5 | 5.2 | Clear |

### Example 7: Screening for Sugar in Formulation

In a 10 mM pH 5.0 acetic acid-sodium acetate buffer system, anti-SOST antibody (Ab-5) solutions were prepared with a protein concentration of 100 mg/mL, a calcium chloride concentration of 4.5 mM, a sugar concentration of 70 mg/mL, a polysorbate 80 concentration of 0.4 mg/mL, and a methionine concentration of 50 mM, containing the following types of sugar, respectively:
(1) sucrose
(2) α,α-trehalose dihydrate

The formulations were added to 2 mL vials at 1.0 mL/vial, and the vials were sealed with film-coated chlorobutyl rubber stoppers and then left to stand at a high temperature (40 °C). The stability of the formulations was evaluated based on their appearance and NR-CE, SEC, and IEC results.

The experimental results are shown in Table 6. The experimental results show that after standing at a high temperature of 40 °C for 7 d, each formulation group had a clear and transparent appearance, and there was no significant difference in their purity results. In addition, when the sucrose concentration was 75 mg/mL, the osmotic pressure of the formulation sample was about 300 mOsm, meaning the formulation was nearly isotonic. 75 mg/mL sucrose was selected for further study.

**Table 6. The stability results for Ab-5 formulations containing different sugars**

| Sugar type | Time | SEC% | | | NR-CE% | IEC% | | |
|---|---|---|---|---|---|---|---|---|
| | | Aggregate | Monomer | Fragment | | Acidic peak | Main peak | Basic peak |
| Sucrose | 0 | 0.5 | 99.5 | 0.0 | 97.7 | 13.6 | 73.9 | 12.4 |
| | 7d | 0.8 | 97.6 | 1.6 | 97.1 | 15.5 | 70.4 | 14.1 |
| α,α-Trehalose dihydrate | 0 | 0.5 | 99.5 | 0.0 | 97.6 | 13.6 | 74.2 | 12.2 |
| | 7d | 0.8 | 97.5 | 1.6 | 97.3 | 15.5 | 71.1 | 13.5 |

### Example 8: Stability Testing of Formulation

In a 10 mM pH 5.0 acetic acid-sodium acetate buffer system, an anti-SOST antibody (Ab-5) solution was prepared with a protein concentration of 100 mg/mL, a calcium chloride concentration of 4.5 mM, a sucrose concentration of 75 mg/mL, a polysorbate 80 concentration of 0.4 mg/mL, and a methionine concentration of 10 mM.

The formulation was added to 1 mL prefilled syringes at 1.0 mL/syringe, and the syringes were sealed with film-coated rubber stoppers and left to stand at 25 °C or 4 °C. The stability of the formulation was evaluated based on its appearance and NR-CE results. The experimental results are shown in Table 7. The experimental results show that the formulation standing either at 25 °C for 3 months or at 4 °C for 18 months yielded stable appearance and NR-CE results, suggesting that the formulation has good stability.

**Table 7. The stability results for the Ab-5 formulation**

| Conditions | NR-CE% | ΔNR-CE% | Appearance |
|---|---|---|---|
| Time 0 | 97.9 | | Clear |
| 25 °C, 3 months | 96.8 | 1.1 | Clear |
| 4 °C, 18 months | 97.9 | 0.0 | Clear |

| | | | |
|---|---|---|---|
| Notes: In the table, ΔNR-CE% refers to the difference between the NR-CE% for the antibody at the start of the experiment (time 0) and the NR-CE% for the antibody at the end of the experiment (25 °C, 3 months; 4 °C, 18 months). | | | |

### Example 9: Stability Testing of High-Concentration Formulation

In a 10 mM pH 5.0 acetic acid-sodium acetate buffer system, an anti-SOST antibody (Ab-5) solution was prepared with a protein concentration of 150 mg/mL, a calcium chloride concentration of 4.5 mM, a sucrose concentration of 75 mg/mL, a polysorbate 80 concentration of 0.4 mg/mL, and a methionine concentration of 10 mM.

The formulation was added to 1 mL prefilled syringes at 1.0 mL/syringe, and the syringes were sealed with film-coated rubber stoppers. The formulation was left to stand at 25 °C or 4 °C (see Table 8 for standing time). The stability of the formulation was evaluated based on its appearance and NR-CE results. The experimental results are shown in Table 8.

**Table 8. The stability results for the high-concentration Ab-5 formulation**

| Conditions | NR-CE% | | | | Appearance |
|---|---|---|---|---|---|
| | Ab-5 main peak | Antibody fragment H1 | Antibody fragment H2 | Antibody fragments H1 + H2 | |
| Time 0 | 97.9 | 0.0 | 0.0 | 0.0 | Clear |
| 25 °C, 6 months | 94.8 | NA | 0.3 | 0.3 | Clear |
| 4 °C, 15 months | 97.8 | 0.0 | 0.0 | 0.0 | Clear |

| | | | | | |
|---|---|---|---|---|---|
| Notes: In the table, for example, "25 °C, 6 months" means standing at 25 °C for 6 months; "NA" means that the content was too low to be determined. | | | | | |

The experimental results show that standing either at 25 °C for 6 months or at 4 °C for 15 months, the formulation containing methionine and the high protein concentration of 150 mg/mL exhibited good stability.

Although specific embodiments of the disclosure have been described above, it will be understood by those skilled in the art that these embodiments are merely illustrative and that many changes or modifications can be made to these embodiments without departing from the principles and spirit of the disclosure. The scope of protection of the disclosure is therefore defined by the appended claims.

## Claims

1. A pharmaceutical composition, comprising an anti-SOST antibody and an antioxidant, wherein the anti-SOST antibody comprises an antibody heavy chain variable region and a light chain variable region, wherein,
the heavy chain variable region comprises a HCDR1 set forth in SEQ ID NO: 3, a HCDR2 set forth in SEQ ID NO: 9 or 4, and a HCDR3 set forth in SEQ ID NO: 5; and the light chain variable region comprises a LCDR1 set forth in SEQ ID NO: 6, a LCDR2 set forth in SEQ ID NO: 7, and a LCDR3 set forth in SEQ ID NO: 8;
preferably, the heavy chain variable region comprises the amino acid sequence set forth in SEQ ID NO: 10, and the light chain variable region comprises the amino acid sequence set forth in SEQ ID NO: 13;
more preferably, the anti-SOST antibody comprises a heavy chain set forth in SEQ ID NO: 22 and a light chain set forth in SEQ ID NO: 25.

2. The pharmaceutical composition according to claim 1, wherein the antioxidant is selected from the group consisting of: methionine, histidine, and arginine;
preferably, the antioxidant is methionine.

3. The pharmaceutical composition according to claim 1 or 2, wherein the concentration of the antioxidant is 1 mM to 100 mM;
preferably, the concentration of the antioxidant is 5 mM to 50 mM;
more preferably, the concentration of the antioxidant is 5 mM to 25 mM;
most preferably, the concentration of the antioxidant is about 10 mM.

4. The pharmaceutical composition according to any of claims 1 to 3, wherein the concentration of the anti-SOST antibody is 1 mg/mL to 300 mg/mL;
preferably, the concentration of the anti-SOST antibody is 50 mg/mL to 150 mg/mL;
more preferably, the concentration of the anti-SOST antibody is 100 mg/mL to 150 mg/mL.

5. The pharmaceutical composition according to any of claims 1 to 4, wherein the pharmaceutical composition has a pH of 4.8 to 5.5;
preferably, the pharmaceutical composition has a pH of 4.8 to 5.2;
more preferably, the pharmaceutical composition has a pH of about 5.0.

6. The pharmaceutical composition according to any of claims 1 to 5, wherein the pharmaceutical composition further comprises a buffer; the buffer is preferably selected from the group consisting of an acetate buffer, a histidine buffer, a phosphate buffer, and a succinate buffer; the buffer is more preferably an acetic acid-sodium acetate buffer; preferably, the concentration of the buffer is 1 mM to 30 mM;
more preferably, the concentration of the buffer is 5 mM to 20 mM;
most preferably, the concentration of the buffer is about 10 mM.

7. The pharmaceutical composition according to any of claims 1 to 6, wherein the pharmaceutical composition further comprises a sugar; the sugar is preferably trehalose or sucrose; the sugar is more preferably α,α-trehalose dihydrate;
preferably, the concentration of the sugar is 40 mg/mL to 95 mg/mL;
more preferably, the concentration of the sugar is 60 mg/mL to 90 mg/mL;
most preferably, the concentration of the sugar is about 75 mg/mL.

8. The pharmaceutical composition according to any of claims 1 to 7, wherein the pharmaceutical composition further comprises a viscosity modifier; the viscosity modifier is preferably selected from the group consisting of a calcium salt, sodium chloride, magnesium chloride, and arginine hydrochloride; the viscosity modifier is more preferably calcium chloride or calcium acetate;
preferably, the concentration of the viscosity modifier is 1 mM to 150 mM;
more preferably, the concentration of the viscosity modifier is 1 mM to 10 mM;
most preferably, the concentration of the viscosity modifier is about 4.5 mM.

9. The pharmaceutical composition according to any of claims 1 to 8, wherein the pharmaceutical composition further comprises a surfactant; the surfactant is preferably polysorbate or poloxamer; the surfactant is more preferably polysorbate 80;
preferably, the concentration of the surfactant is 0.02 mg/mL to 0.8 mg/mL;
more preferably, the concentration of the surfactant is 0.3 mg/mL to 0.6 mg/mL;
most preferably, the concentration of the surfactant is about 0.4 mg/mL.

10. The pharmaceutical composition according to any of claims 1 to 9, wherein the pharmaceutical composition comprises:
(a) 1 mg/mL to 300 mg/mL said anti-SOST antibody, (b) 1 mM to 100 mM methionine, histidine, or arginine, (c) 40 mg/mL to 95 mg/mL trehalose or sucrose, (d) 1 mM to 30 mM acetate buffer or histidine buffer, (e) 1 mM to 20 mM calcium salt, and (f) 0.02 mg/mL to 0.8 mg/mL polysorbate, and the pharmaceutical composition has a pH of 4.8 to 5.5;
preferably, the pharmaceutical composition comprises:
(a) 50 mg/mL to 150 mg/mL anti-SOST antibody comprising a heavy chain of SEQ ID NO: 22 and a light chain of SEQ ID NO: 25, (b) 5 mM to 25 mM methionine, (c) 60 mg/mL to 90 mg/mL trehalose or sucrose, (d) 5 mM to 20 mM acetate buffer, (e) 1 mM to 10 mM calcium chloride, and (f) 0.3 mg/mL to 0.6 mg/mL polysorbate 80, and the pharmaceutical composition has a pH of 4.8 to 5.2;
more preferably, the pharmaceutical composition comprises:
(a) about 100 mg/mL anti-SOST antibody comprising a heavy chain of SEQ ID NO: 22 and a light chain of SEQ ID NO: 25, (b) about 10 mM methionine, (c) about 75 mg/mL trehalose or sucrose, (d) 10 mM to 20 mM acetic acid-sodium acetate buffer, (e) about 4.5 mM calcium chloride, and (f) about 0.4 mg/mL polysorbate 80, and the pharmaceutical composition has a pH of about 5.0;
most preferably, the pharmaceutical composition comprises:
(a) about 100 mg/mL anti-SOST antibody comprising a heavy chain of SEQ ID NO: 22 and a light chain of SEQ ID NO: 25, (b) about 10 mM methionine, (c) about 75 mg/mL sucrose, (d) about 10 mM acetic acid-sodium acetate buffer, (e) about 4.5 mM calcium chloride, and (f) about 0.4 mg/mL polysorbate 80, and the pharmaceutical composition has a pH of about 5.0.

11. The pharmaceutical composition according to any of claims 1 to 10, wherein
the pharmaceutical composition comprises an intact anti-SOST antibody but does not comprise a broken anti-SOST antibody; a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 27; or
the pharmaceutical composition comprises an intact anti-SOST antibody and a broken anti-SOST antibody; a heavy chain of the intact anti-SOST antibody comprises the amino acid sequence set forth in SEQ ID NO: 22, and a light chain comprises the amino acid sequence set forth in SEQ ID NO: 25; the amino acid sequence of a heavy chain of the broken anti-SOST antibody is set forth in SEQ ID NO: 27, and the proportion of the broken anti-SOST antibody to the total antibody amount is no more than 25%; preferably, the proportion of the broken anti-SOST antibody to the total antibody amount is no more than 16%; more preferably, the proportion of the broken anti-SOST antibody to the total antibody amount is no more than 10%.

12. A lyophilized formulation, obtained by lyophilizing the pharmaceutical composition according to any of claims 1 to 11.

13. A method for treating an SOST-related disease or condition, wherein the method comprises administering to a subject in need thereof a therapeutically effective amount of:
(A) the pharmaceutical composition according to any of claims 1 to 11, or
(B) the lyophilized formulation according to claim 12;
preferably, the SOST-related disease or condition is selected from the group consisting of a disease or disorder of osteoporosis, osteopenia or osteoarthritis, rheumatoid arthritis, periodontal disease, or multiple myeloma;
more preferably, the SOST-related disease or condition is osteoporosis.
